# EUROPEAN PATENT APPLICATION

(11) **EP 3 800 531 A1**
(43) Date of publication of application: **07.04.2021**
(21) Application number: 20199798.8
(22) Date of filing: 02.10.2020
(51) Int. Cl.: G06F 3/01

(54) **DEVICE FOR HUMAN-MACHINE INTERFACING BY MEANS OF BRAIN SIGNALS AND ASSOCIATED INTERFACING METHOD**

(30) Priority: 04.10.2019 IT 201900017990
(71) Applicant: HRH-BRK Societa' a Responsabilita' Limitata Semplificata, 22066 Mariano Comense (CO) (IT)
(72) Inventor: URBANO, Nicola Gianmarco, 22066 Mariano Comense (CO) (IT)
(74) Representative: PGA S.p.A.

(57) **Abstract**

Human-machine interface device (1), comprising:
- at least one input (12) configured to receive at least one biological electrical signal (S), in particular coming from at least the brain of at least one subject (13),
- an electronic circuit (11), electrically connected to the input (12) configured to electronically process the biological electrical signal (S),
- at least one I/O terminal (14), operatively connected to the electronic circuit (11) and configured at least to transmit a data signal (30) comprising the biological electrical signal (S) processed by the electronic circuit (11) and/or based on the biological electrical signal (S) processed by the electronic circuit (11) toward a machine (40), through a fieldbus (20), and/or configured to receive a data signal (30) from said fieldbus (20),
said human-machine interface device (1) being configured to transmit the data signal (30) and to receive further data signals (30) on/from a fieldbus, said electronic circuit (11) and said I/O terminal (14) being configured at least to process and retransmit the biological electric signal (S) substantially in real time.

## Description

### FIELD OF THE INVENTION

The present invention relates to the field of brain computer interface (BCI) devices and in detail it relates to a human-machine interface device through brain signals and to a method of interfacing between a human and an associated machine.

### STATE OF THE ART

BCIs are interface devices that exploit brain waves. The field of such interface devices is known both in the experimental field and in the academic field. The basic idea is to control machines using brain interfaces.

The Applicant points out that the use of increasingly complex machines requires a large amount of controls for their normal operation and points out that in particular some machines may require control signals coming from controls controlled by subjects geographically located in different positions.

The Applicant also points out that, especially when the machines are fast, serious operating limits already exist with traditional commands when the subjects are placed in a remote position with respect to the machine, due to latencies which often cause operating limits during the control.

Current Ethernet control systems allow to connect data processing units remote with respect to each other, and also machines remote with respect to each other; Ethernet systems are not of deterministic type, and i.e. they do not allow to guarantee predefined reaction times, for example due to sporadic collisions caused by non-synchronised master devices that intend to access the common driver.

Interface devices using data buses are known from document WO 2019/079378 and from document Jinhua Zhang ET AL: "An EEG/EMG/EOG-Based Multimodal Human- Machine Interface to Real-Time Control of a Soft Robot Hand", Frontiers in Neurorobotics, vol. 13, 29 March 2019.

The object of the present disclosure is to describe a human-machine interface which allows subjects to be integrated into complex systems, and which allows to analyse stimuli not only related to the BCI but to the whole human body.

### SUMMARY

The object of the present disclosure will now be described according to some major aspects, which can be combined to each other and/or combined with portions of the following detailed description and/or with the attached claims. If combinability or dependencies between aspects are defined, they shall be deemed as non-limiting.

According to a first aspect, herein described is a human-machine interface device (1), comprising:
- at least one input (12) configured to receive at least one biological electrical signal (S), optionally coming from at least the brain of at least one subject (13),
- an electronic circuit (11), electrically connected to the input (12) configured to electronically process the biological electrical signal (S),
- at least one I/O terminal (14), operatively connected to the electronic circuit (11) configured at least to transmit a data signal (30) comprising the biological electrical signal (S) processed by the electronic circuit (11) and/or based on the biological electrical signal (S) processed by the electronic circuit (11) toward a machine (40), through a fieldbus (20), and/or configured to receive a data signal (30) from said fieldbus (20),
said human-machine interface device (1) being configured to transmit the data signal (30) and to receive further data signals (30) on/from a fieldbus, said electronic circuit (11) and said I/O terminal (14) being configured at least to process and retransmit the biological electric signal (S) substantially in real time.

According to a further non-limiting aspect, combinable with one or more of the present aspects, said electronic circuit (11) and said I/O terminal (14) are configured at least to keep a deterministic component and/or to keep a predetermined speed in the transmission of the signal on the fieldbus (20).

According to a further aspect, herein described is a human-machine interface device, comprising:
- at least one input (12) configured to receive at least one biological electrical signal (S), optionally coming from at least the brain of at least one subject (13),
- an electronic circuit (11), electrically connected to the input (12) configured to electronically process the biological electrical signal (S),
- at least one I/O terminal (14), operatively connected with the electronic circuit (11) and configured at least to transmit a data signal (30) comprising the biological electrical signal (S) processed by the electronic circuit (11) and/or based on the biological electrical signal (S) processed by the electronic circuit (11) towards a machine, through a fieldbus (20), and/or configured to receive a data signal (30) from said fieldbus (20),
said human-machine interface device (1) being a device configured to transmit the data signal (30) comprising at least part of said biological electrical signal (S) and to receive a data signal (30) on/from a fieldbus, said electronic circuit (11) and said I/O terminal (14) being configured at least to keep a deterministic component and/or to keep a predetermined speed in the transmission of the data signal (30), in particular of the respective data signals, on the fieldbus (20).

According to a further non-limiting aspect, the deterministic component is such that the transceive of data signals on said fieldbus (20) occurs at pre-set time intervals.

According to a further non-limiting aspect, combinable with one or more of the present aspects, the fieldbus is a data bus specifically designed to allow the distributed control of a machine (40) by several electronic devices, in particular by means of a serial data transceive.

According to a further non-limiting aspect, combinable with one or more of the present aspects, the fieldbus is an EtherCAT bus, or it is a Profinet bus, or it is a Sercos III bus, or it is a Powerlink bus, or it is an EtherNet/IP bus.

According to a further non-limiting aspect, combinable with one or more of the present aspects, the biological electrical signal (S) is, or comprises, at least one of the signals of the following list: an electroencephalographic signal, or an electrocardiographic signal, a sleep study monitoring signal, a bispectral analysis signal, a signal of evoked potentials.

According to a further non-limiting aspect, combinable with one or more of the present aspects, the human-machine interface device is configured to operate simultaneously with further human-machine interface devices.

According to a further non-limiting aspect combinable with one or more of the present aspects, the human-machine interface device (1) is configured to receive, in particular to sample and/or quantise, said biological electric signal (S) on said input (12), optionally for a predetermined and finite period of time, in a first reception mode and based on a distributed clock signal, transmitted on the fieldbus (20), and wherein in said first reception mode the human-machine interface device (1) is configured to compensate for signal propagation delays in said fieldbus (20), and/or it is configured to receive, and/or quantise and/or sample the biological electrical signal (S) at the input (12), optionally for a predetermined and finite period of time, in a second reception mode, by generating an interrupt signal caused by the reception of a data signal (30) on the I/O terminal (14).

According to a further non-limiting aspect, combinable with one or more of the present aspects, the human-machine interface device (1) is configured to operate in a first distributed clock mode in which the transmission of data signals (30) on the I/O terminal (14) and at least the reception, and/or quantisation and/or sampling of the biological electrical signal (S) on the input (12) are performed at predefined time instants based on a clock signal.

According to a further non-limiting aspect, combinable with one or more of the present aspects, the human-machine interface device is configured to automatically activate, following the generation of said interrupt signal, a reception and/or electronic sampling of the biological electrical signal (S) on said input (12), and/or the human-machine interface device (1) is configured to receive and/or sample the biological electrical signal (S) in at least one predetermined instant of time at the generation of said interrupt signal and/or determined by the generation of said interrupt signal.

According to a further non-limiting aspect, combinable with one or more of the present aspects, the human-machine interface device (1) is a slave-type device configured to receive a synchronisation signal from a master controller (500) transmitting the synchronisation signal on the same fieldbus (20) to which the human-machine interface device (1) accesses through the I/O terminal (14).

According to a further non-limiting aspect, combinable with one or more of the present aspects, the human-machine interface device (1) is a slave device, configured at least to transmit on said fieldbus (20) data and/or signals directly directed to a master controller (500).

According to a further non-limiting aspect, combinable with one or more of the present aspects, the human-machine interface device (1) is configured to sample data signals (30) and/or biological electrical signals (S) at a frequency greater than or equal to 1.0kHz, preferably greater than or equal to 1.5kHz, or even more preferably greater than or equal to 2kHz.

According to a further non-limiting aspect, combinable with one or more of the present aspects, the human-machine interface device is configured to sample data signals (30) and/or biological electrical signals (S) at a frequency greater than or equal to 5 kHz, preferably greater than or equal to 10 kHz, preferably greater than or equal to 15 kHz, preferably up to 20 kHz.

According to a further non-limiting aspect, combinable with one or more of the present aspects, the electronic circuit (11) is configured to sample and/or quantise said biological electrical signal (S) on a number of bits ≥12, preferably ≥16, even more preferably ≥20, even more preferably equal to 24 bits.

According to a further non-limiting aspect, combinable with one or more of the present aspects, said data signal (30) comprises a biological electrical signal (S) quantised according to the preceding aspect and/or comprises a biological electrical signal (S) quantised according to a scale of precision greater than or equal to 0.5 µV.

According to a further non-limiting aspect, combinable with one or more of the present aspects, said data signal (30) transmission deterministic and/or speed component is established following the reception of said synchronisation signal.

According to a further non-limiting aspect, combinable with one or more of the present aspects, the human-machine interface device (1) is configured to retransmit the data signal (30) received on the I/O terminal (14) again on the fieldbus (20) through the I/O terminal (14); said human-machine interface device (1) being configured to automatically generate an interrupt signal, in particular both event and time-based, upon receiving a given data signal (30) on the I/O terminal (14).

According to a further non-limiting aspect, combinable with one or more of the present aspects, said human-machine interface device (1) is configured to automatically generate an interrupt signal, in particular both event and time-based, in particular, upon receiving a data signal containing a synchronisation EtherCAT frame.

According to a further non-limiting aspect, combinable with one or more of the present aspects, the human-machine interface device (1) comprises a plurality of inputs (12), optionally in number equal to 8, or 9, or 10, and it is configured to simultaneously sample biological electrical signals (S) present on the plurality of inputs (12).

According to a further non-limiting aspect, combinable with one or more of the present aspects, the electronic circuit (11) comprises at least one first operating unit (110) for managing human-machine interface biological signals, connected to the input (12) and/or comprising the input (12).

According to a further non-limiting aspect, combinable with one or more of the present aspects, the first operating unit (110) is configured to operate in a first single-end mode or in a second and different differential mode and/or to receive the biological electrical signal (S) on the input (12) in a first single-end mode or in a second and different differential mode, and/or the human-machine interface device (1) is configured to operate in a first single-end mode or in a second and different differential mode.

According to a further non-limiting aspect combinable with one or more of the present aspects, in the single-end mode, the first operating unit (110) is configured to report the biological electric signal (S) received on the input (12) to a reference signal, optionally a mass signal, which is defined and/or available inside the human-machine interface device (1), and in the differential mode the first operating unit (110), is configured to report the reference signal, optionally said mass signal, to a signal taken outside the human-machine interface device (1).

According to a further non-limiting aspect, combinable with one or more of the present aspects, the human-machine interface device (1), in particular, the first operating unit (110), comprises a switch and/or a switch configured at least to allow the alternate selection between said first single-end mode and said second differential mode.

According to a further non-limiting aspect, combinable with one or more of the present aspects, the electronic circuit (11) comprises a second operating unit (120), operatively connected to the first operating unit (110), said second operating unit (120) being configured to manage the specific operation of the human-machine interface device (1).

According to a further non-limiting aspect, combinable with one or more of the present aspects, the electronic circuit (11) comprises a third operating unit (130), operatively connected at least to the second operating unit (120), said third operating unit (130) being configured to supervise the control of the data signals passing through said fieldbus (20).

According to a further non-limiting aspect, combinable with one or more of the present aspects, the third operating unit (130) comprises and/or it is connected, with said I/O terminal (14).

According to a further non-limiting aspect, combinable with one or more of the present aspects, the human-machine interface device (1) comprises a data acquisition subsystem (470) configured to operate as an interface stage towards the plurality of inputs (12).

According to a further non-limiting aspect, combinable with one or more of the present aspects, the human-machine interface device (1) comprises a logic control unit (480) operatively connected to the data acquisition subsystem (470), optionally electrically connected to the data acquisition subsystem (470).

According to a further non-limiting aspect, combinable with one or more of the present aspects, the human-machine interface device (1), in particular the electronic circuit (11), comprises a fieldbus interface subsystem (490), optionally an EtherCat interface subsystem, configured to manage a serial data flow from and toward the I/O terminal (14).

According to a further non-limiting aspect, combinable with one or more of the present aspects, the input (12) and/or the I/O terminal (14) comprise connectors of the standard type, optionally of the M8 and/or M12 type.

According to a further non-limiting aspect, combinable with one or more of the present aspects, the logic control unit (480) is configured to manage the synchronisation of the data acquisition subsystem (470) and of the fieldbus interface subsystem (490) and/or to manage the synchronisation between the data acquisition subsystem (470) and the fieldbus interface subsystem (490).

According to a further non-limiting aspect, combinable with one or more of the present aspects, the logic control unit (480) comprises a microprocessor, and a real-time clock (RTC) module (428) configured to manage the programming of the real-time actions performed by the logic control unit (480) by defining, for each action, priority over other actions and/or by defining, for each action, a predefined run time.

According to a further non-limiting aspect, combinable with one or more of the present aspects, the logic control unit (480) comprises a memory operatively connected at least to said microprocessor.

According to a further non-limiting aspect, combinable with one or more of the present aspects, the human-machine interface device (1) comprises a plurality of data acquisition subsystems (470) configured to operate in a simultaneous and/or parallel and/or stack fashion with respect to each other.

According to a further non-limiting aspect, combinable with one or more of the present aspects, the logic control unit (480) is configured to communicate in a parallel and/or simultaneous fashion with said plurality of data acquisition subsystems (470).

According to a further non-limiting aspect, combinable with one or more of the present aspects, the fieldbus interface subsystem (490) comprises a network interface controller (457) operatively connected with the I/O terminal (14).

According to a further non-limiting aspect, combinable with one or more of the present aspects, the I/O terminal (14) comprises a connector of the RJ45 type and/or a connector of the M8 or M12 type.

According to a further non-limiting aspect, combinable with one or more of the present aspects, the human-machine interface device (1) comprises a temperature sensor (425) operatively connected to the processor (420) and configured to detect a temperature present in real time at least inside the human-machine interface device (1).

According to a further non-limiting aspect, combinable with one or more of the present aspects, the logic control unit (480) comprises at least one inner bus selected from the plurality of buses of the following list: SPI, QSPI, I2C, or 8/16/32-bit parallel bus communication.

According to a further non-limiting aspect, combinable with one or more of the present aspects, the fieldbus interface subsystem (490) comprises a machine learning module (424) operatively connected with the processor (420).

According to a further non-limiting aspect, combinable with one or more of the present aspects, the processor (420) is configured to process the biological electrical signals (S) received through the input (12) through said machine learning module (424) and/or through a digital signal processing module part of the logic control unit (480).

According to a further non-limiting aspect, combinable with one or more of the present aspects, the fieldbus interface subsystem (490) comprises a mailbox (453), configured to manage an acyclic-type data transceive; optionally said mailbox (453) carrying out said transceive according to at least one of the standards selected from the present list: CANOpen over EtherCAT, File over EtherCAT, Ethernet over Ethercat, Safety over EtherCAT, Vendor Specific.

According to a further non-limiting aspect, combinable with one or more of the present aspects, the fieldbus interface subsystem (490) comprises a distributed clock synchronisation module (450) configured to receive a clock signal from a master controller (500) external to the human-machine interface device (1), optionally to receive a clock signal with a synchronisation precision between several human-machine interface devices (1) in the order of the nanosecond.

According to a further non-limiting aspect, combinable with one or more of the present aspects, the human-machine interface device (1) comprises a casing from which the following are faced: the I/O terminal (14) and/or at least one input (12).

According to a further non-limiting aspect, combinable with one or more of the present aspects, the casing is a casing according to IEC 70-1 standard and/or it is a waterproof casing, and/or is a dust-proof casing.

According to a further aspect herein provided is a human-machine interface system; said system comprising:
- a master controller (500), configured to at least control a plurality of human-machine interface devices (1) according to one or more of the preceding aspects,
- at least one human-machine interface device (1) according to one or more of the preceding aspects,
- a fieldbus (20), operatively accessed by the master controller (500) and by the at least one human-machine interface device (1),
wherein the assembly formed by the master controller (500) and by the at least one human-machine interface device (1) form a network, and wherein the master controller (500) is configured to synchronise said network, configuring it to carry out the transceive substantially in real time and/or with deterministic component and substantially predefined speed.

According to a further non-limiting aspect, combinable with one or more of the present aspects, the system is configured and/or designed to compensate for propagation delays of signals transmitted on the fieldbus (20), in particular to compensate for propagation delays of data signals (30) transmitted on said fieldbus, optionally by means of a distributed clock signal such to cause a reception and/or sampling and/or quantisation of biological electric signals (S) on the inputs (12) of respective human-machine interface devices (1) in substantial temporal simultaneity.

According to a further non-limiting aspect, combinable with one or more of the present aspects, said master controller (500) is configured to send on said fieldbus (20) a synchronisation and/or distributed clock signal configured to allow the synchronisation of at least one human-machine interface device (1), optionally a plurality of human-machine interface devices (1), with a synchronisation precision in the order of the nanosecond.

According to a further non-limiting aspect, combinable with one or more of the present aspects, said master controller (500) is configured to send on said fieldbus (20) a synchronisation and/or distributed clock signal configured to allow the synchronisation of at least one human-machine interface device (1), optionally, a plurality of human-machine interface devices (1), such that the synchronisation precision between the human-machine interface devices (1) provided through the synchronisation signal and/or the distributed clock is independent of the number of human-machine interface devices (1) connected to the fieldbus (20).

According to a further non-limiting aspect, combinable with one or more of the present aspects, the master controller (500) comprises a microprocessor (542), optionally a single-core or multicore microprocessor, and/or it comprises a PLC and/or an Intel, ARM or AMD technology processor.

According to a further non-limiting aspect, combinable with one or more of the present aspects, the system is a distributed clock system and the master controller (500) comprises a clock signal distribution module (543) configured to cause - in use - transmission of said synchronisation and/or clock signal distributed on the fieldbus toward one or more human-machine interface devices (1) connected thereto, said clock signal distribution module (543) being operatively connected to said processor (542).

According to a further non-limiting aspect, combinable with one or more of the present aspects, the system comprises a plurality of human-machine interface devices (1) operating substantially simultaneously and/or concurrently, in particular by means of a transmission of said data signal (30), on said network and/or on said fieldbus (20).

According to a further non-limiting aspect, combinable with one or more of the present aspects, the master controller (500) comprises a memory on which an operating system is loaded, said operating system being optionally a Linux, or Windows, or Android system, or an embedded operating system, or an open source operating system, said operating system being characterised by a realtime extension, in particular a real-time extension configured to manage a fieldbus network, in particular an EtherCAT network, optionally in which the extension is configured to keep the determinism of the application.

According to a further non-limiting aspect, combinable with one or more of the present aspects, the master controller (500) comprises a memory in which a plurality of communication profiles for managing the communication protocols between the peripherals operatively connected to said fieldbus (20) are specified.

According to a further non-limiting aspect, combinable with one or more of the present aspects, the profiles comprise motion management profiles, wherein the data signals (30) are motion control signals, optionally wherein said motion management profiles are according to the DS402 standard, and/or input/output data management profiles, optionally wherein said input/output data management profiles are according to the DS301 standard.

According to a further non-limiting aspect, combinable with one or more of the present aspects, the system is configured to operate in a first distributed clock mode wherein the transmission of data signals (30) on the I/O terminal (14) and at least the reception, and/or quantisation and/or sampling of the biological electrical signal (S) on the input (12) are performed at predefined time instants based on a clock signal, and in which said predefined time instants are substantially the same for each human-machine interface device (1) operatively connected on said fieldbus (20).

According to a further aspect, herein described is a human-machine interface method, in particular by means of a human-machine interface device (1), said method comprising:
- a step of receiving, on an input (12) of the human-machine interface device (1), at least one biological electrical signal (S), in particular coming from at least the brain of a subject (13),
- a processing step, wherein the biological electrical signal (S) received on the input (12) is electronically processed by an electronic circuit (11), electrically connected to the input (12),
- a step of transmitting a data signal (30) comprising the biological electrical signal (S) processed by the electronic circuit (11) and/or based on the biological electrical signal (S) processed by the electronic circuit (11), wherein in said transmission step the data signal (30) is transmitted, by means of an I/O terminal (14) of said human-machine interface device (1), directly on a fieldbus (20), in particular an EtherCAT-type fieldbus, so as to be directed towards a machine (40),
   wherein the processing of said data signal (30) occurs substantially in real time, and wherein as a result of said transmission step the machine (40) is controlled performing operations related to said data signal (30) transmitted on the fieldbus (20).

According to a further aspect, herein described is a human-machine interface method, in particular by means of a human-machine interface device (1), said method comprising:
- a step of receiving, on an input (12) of the human-machine interface device (1), at least one biological electrical signal (S), in particular coming from at least the brain of a subject (13),
- a processing step, wherein the biological electrical signal (S) received on the input (12) is electronically processed by an electronic circuit (11), electrically connected to the input (12),
- a step of transmitting a data signal (30) comprising the biological electrical signal (S) processed by the electronic circuit (11) and/or based on the biological electrical signal (S) processed by the electronic circuit (11), wherein in said transmission step the data signal (30) is transmitted, by means of an I/O terminal (14) of said human-machine interface device (1), directly on a fieldbus (20) so as to be directed towards said machine,
   wherein the processing of said data signal (30) occurs with the maintenance of a deterministic and/or speed component in the transmission of the signal on the fieldbus (20),
   and wherein as a result of said transmission step the machine (40) is controlled, performing operations related to said data signal (30) transmitted on the fieldbus (20).

According to a further non-limiting aspect, the human-machine interface device (1) of the two preceding aspects is a human-machine device (1) according to one or more of the aspects described herein.

According to a further non-limiting aspect, combinable with one or more of the present aspects, the method comprises a step of positioning an electric sensor (416) on the body of said subject (13) for acquiring the biological electric signal (S).

According to a further non-limiting aspect, combinable with one or more of the present aspects, the method comprises establishing a logic connection between a master controller (500), configured at least to control a plurality of human-machine interface devices (1) according to one or more of the preceding aspects, and at least one human-machine interface device (1), wherein following the establishment of said logic connection, the master controller (500) and the human-machine interface device (1) form a network interconnected by means of said fieldbus (20); said method comprising the running of an electronic synchronisation of the network, performed by the master controller (500), following which the network is configured to operate to transceive substantially in real-time and/or with deterministic component and substantially predefined speed.

According to a further non-limiting aspect, combinable with one or more of the present aspects, following synchronisation, data signals (30) are transmitted on said network and/or on said fieldbus (20), optionally in the form of frames, at constant and deterministic time intervals.

According to a further non-limiting aspect, combinable with one or more of the present aspects, the step of receiving the biological electrical signal (S) comprises receiving and/or sampling and/or quantising said biological electrical signal (S) on said input (12), optionally for a predetermined and finite period of time, in a first reception mode and based on a distributed clock signal, transmitted on the fieldbus (20), and wherein in said first reception mode the human-machine interface device (1) is configured to compensate for signal propagation delays in said fieldbus (20), and/or comprises receiving, and/or quantising and/or sampling, the biological electric signal (S) at the input (12), optionally for a predetermined and finite period of time, in a second reception mode, by generating an interrupt signal caused by the reception of a data signal (30) on the I/O terminal (14).

According to a further non-limiting aspect, combinable with one or more of the present aspects, the method comprises a step of distributing a clock signal on the network and/or on the fieldbus (20), in order to electronically synchronise a plurality of human-machine interface devices (1) connected thereto.

According to a further non-limiting aspect, combinable with one or more of the present aspects, the clock distribution step occurs by configuring and/or programming the master controller (500), and in particular a clock signal distribution module (543) thereof, for transmitting a synchronisation and/or clock signal distributed on the fieldbus (20) toward one or more human-machine interface devices (1) connected thereto.

According to a further non-limiting aspect, combinable with one or more of the present aspects, the method comprises a step for the electronic management of response to stimuli sent to a plurality of users, each transmitting a biological electric signal (S) thereof toward a respective human-machine interface device (1), in turn transmitting said data signal (30) on the network and/or on said fieldbus (20).

According to a further non-limiting aspect, combinable with one or more of the present aspects, the reception of the biological electric signal (S) on the first input of the human-machine interface device (1) is a reception performed in single-end mode or, alternatively, a reception performed in differential mode.

According to a further non-limiting aspect, in the single-end mode, the biological electric signal (S) received on the input (12) is reported to a reference signal, optionally a mass signal, which is defined and/or available inside the human-machine interface device (1), and in the differential mode, the reference signal, optionally said mass signal, is a signal taken outside the human-machine interface device (1).

According to a further non-limiting aspect, combinable with one or more of the present aspects, there is a selection step in which an operator acts on a selector and/or switch installed on said human-machine interface device (1), in particular on a first operating unit (110) of the human-machine interface device (1) configured to manage the human-machine interface signals (1) coming from the subject (13), wherein the action on said selector and/or switch causes the alternative selection between the single-end mode and the differential mode.

According to a further non-limiting aspect, combinable with one or more of the present aspects, the method comprises applying a bias signal to the biological electric signal (S) upon performing and/or following said reception step on said input (12).

### BRIEF DESCRIPTION OF THE DRAWINGS

The object of the present disclosure will now be described in preferred and non-limiting embodiments described with the help of the attached drawings, wherein:
- figure 1 illustrates a block diagram representing in a simplified manner a fieldbus network to which a master unit of the EtherCAT type and a plurality of human-machine interface devices are connected;
- figure 2 illustrates a simplified block diagram of a human-machine interface device which subject of the present disclosure;
- figure 3 illustrates a block diagram showing that different human-machine interface devices can be used to intercept biological signals coming from different areas of the body of a subject;
- figure 4 illustrates a detailed diagram of the human-machine interface device subject of the present disclosure;
- figure 5 illustrates a detailed diagram of a master controller part of the system subject of the present disclosure;
- figure 6 illustrates a computing macrosystem comprising several systems according to the present disclosure;
- figure 7 illustrates a simplified flow chart of a data acquisition cycle by means of the system subject of the present disclosure.

### DETAILED DESCRIPTION

Forming an object of the present disclosure is a human-machine interface device which is generally indicated in its entirety with reference numeral 1 in the attached figures.

The device subject of the present disclosure, which provides a neural interface, is designed to fill the void existing in the prior art especially so as to be able to analyse biological signals coming from one or more users in a precise and deterministic manner, so as to be able to effectively control a plurality of machines, even by means of the simultaneous interaction of several users. In particular, a neural interface system of the master-slave type which allows to control two or more machines simultaneously without the need for dedicated synchronisation between one machine and the other will be described in the present disclosure.

Furthermore, the human-machine interface device subject of the present disclosure allows the use of EtherCAT for the acquisition of medical signals.

Furthermore, the human-machine interface device, the interfacing system and method subject of the present disclosure allow the modularisation of the acquisition probes. Such probes can be arranged on different human-machine interface devices described herein, even in different spatial positions.

In particular, should different human-machine interface devices be connected to each other by means of an interface of the EtherCAT type, and in particular when connected by means of an optical fibre, it is possible to have human-machine interfaces arranged even one kilometre from each other.

Furthermore, through the human-machine interface device, the interfacing system and method described herein, it is possible to obtain multi-user signal analysis and no longer solely dedicated to individual patients. This paves way to the possibility to conduct tests and trials with teams of people who are managed simultaneously.

The human-machine interface device and the system described herein are compatible with the standards currently in use for industrial machines, and thus they do not need to implement further dedicated interfaces, especially on the machine side. In particular, the human-machine interface device described herein, as well as the interfacing system and method, allow compatibility with the Industry 4.0 standard.

Thanks to the fact that the device subject of the present disclosure provides an interface compliant with different real-time fieldbus profiles (in particular, in the description, EtherCAT) and with Industry 4.0, it is possible to find on the market several suppliers justifying a value of the costs nominally lower than the current implementations.

Transceive speeds and time instants for transmitting predictable signals (deterministic component) with frequency higher than one kHz, being able to control fast machines too with extreme accuracy, are obtained through the human-machine interface device described herein, as well through the system and method subject of the present disclosure. In particular, such frequency may be greater than or equal to 1 kHz, or greater than or equal to 1.5 kHz, or greater than or equal to 2 kHz. In a preferred and non-limiting embodiment, the transceive speed may also occur at frequencies greater than or equal to 10 kHz, or more preferably greater than or equal to 15 kHz, up to reaching frequencies substantially close to 20 kHz.

The human-machine interface device subject of the present disclosure is compatible without any need for adaptations with any master module, in particular using the same fieldbus profile as the device itself.

The human-machine interface device, the system subject of the present disclosure, are compatible with software for the management and control of neural networks and machine learning. The method described herein allows not only to interface a subject with a machine, but also to implement algorithms for controlling neural networks and machine learning.

The human-machine interface device, the system and the method subject of the present disclosure are applicable to persons with limited or absent motion dynamics, for example for the study and analysis of neural processes.

The human-machine interface device and the system subject of the present disclosure interfaces toward external devices provided with connectors with a pinout that is compatible with current Arduino systems.

Preferably operating according to an EtherCAT protocol, the device subject of the present disclosure complies with the certified interfacing specifications. The latter regard both the profiles and operating modes.

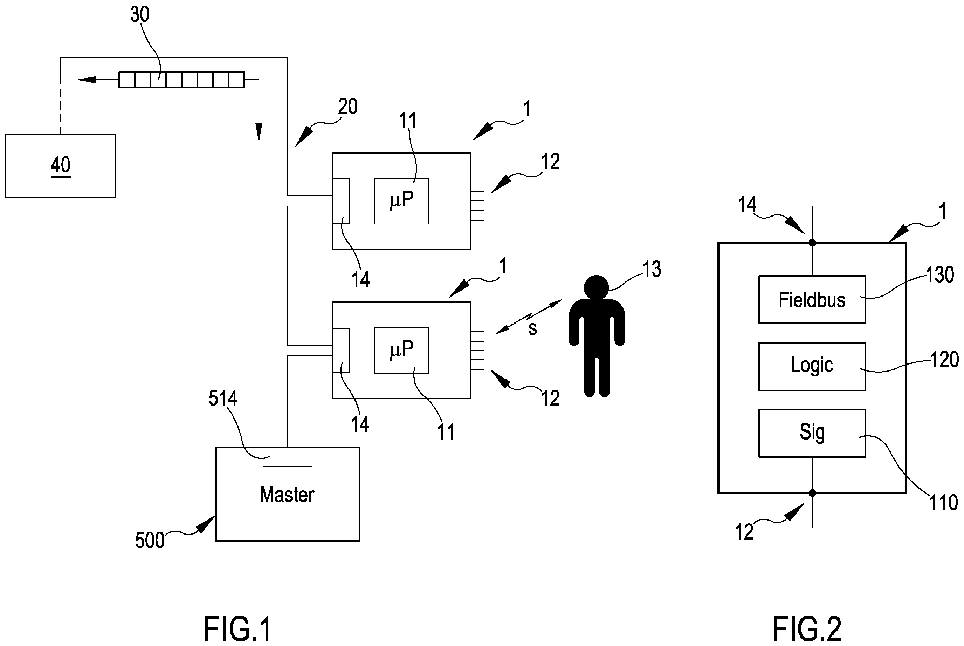

As shown in figure 1, the system subject of the present disclosure comprises a master controller 500 which is operatively connected by means of a fieldbus 20 to at least one, preferably a plurality of, human-machine interface devices 1. In a non-limiting embodiment such as the one illustrated in figure 1 each human-machine interface device 1 is connected to the adjacent one by means of a serial connection.

For the purposes of the present disclosure, the expression "fieldbus" is used to indicate a bus configured for, and specifically designated to allow distributed control of a machine 40 by a plurality of electronic devices positioned in distinct positions with respect to each other; such distinct positions taken by said electronic devices can reach the order of magnitude of the kilometres of distance. From the physical point of view, the fieldbus is a bundle of electric conductors; such configuration shall not be intended in a limiting manner, since it is possible to have optical fibre fieldbuses, or at least partially made of optical fibre. For the purposes of this disclosure, the data is transmitted serially on the fieldbus.

In a preferred embodiment, the fieldbus 20 is an EtherCAT bus. EtherCAT exploits the transmission on the fieldbus 20 of a data signal 30 of the Ethernet frame type, which - through a distributed clock - is transmitted in a predictable manner thus allowing the maintenance of the deterministic component.

For the purposes of this disclosure, the expression "operatively connected" is used to indicate a connection between one element and another element, of a logical and/or physical type, in which data and/or signals are exchanged. In a particular non-limiting embodiment, the operative connection is an electrical connection; alternatively, and still in a non-limiting manner, the connection is an optical connection.

The fieldbus 20 is operatively connected to a machine 40, which can be an industrial machine or any other. Electrical or optical signals may pass through the fieldbus 20, equivalently.

Each human-machine interface device 1, comprises:
- at least one input 12 configured to receive at least one biological electrical signal (S), in particular coming from at least the brain of a subject 13,
- an electronic circuit 11, electrically connected to the input 12 configured to electronically process the biological electrical signal S,
- at least one I/O terminal 14 configured at least to transmit a data signal 30 comprising the biological electrical signal S processed by the electronic circuit 11 and/or based on the biological electrical signal S processed by the electronic circuit 11 toward a machine 40, through a fieldbus 20, and/or configured to receive a data signal 30 from said fieldbus 20.

It is pointed out here that the data signal 30 that passes through the fieldbus 20 does not necessarily always have to have at least part of the biological electrical signal S.

The human-machine interface device 1 described herein is a device configured to transmit the data signal 30 therein including at least part of the biological electrical signal S and to receive a data signal 30 on/from a fieldbus, and the electronic circuit 11 and/or said I/O terminal 14 are configured at least for processing and transceiving the biological electrical signal S substantially in real time. The aforementioned deterministic component is substantially kept through the cooperation of the electric circuit 11 and the I/O terminal 14.

As shown in figure 2, the electronic circuit 11 of each human-machine interface device 1 consists of three main operating units identified with the reference numerals 110, 120, 130; a first operating unit is the one for managing BCI signals measured by means of sensors connected to the body of the subject 13, and therefore also comprises the input 12. In a particular embodiment, the first operating unit 110 may operate in single-end mode or differential mode. In detail, in the single-end mode, the electrical signal received by the sensors on the input 12 is reported to a ground signal, or in any case a reference signal, preferably but not limited to 0V, which is defined and available inside the device described herein. In the differential mode, on the other hand, the mass signal, or in any case the aforementioned reference signal, is not internal and it must be taken outside the device subject of the present disclosure. Single-end mode offers the advantage of simplifying wiring with probes outside the device described herein. A selector or switch, a dip-switch which through external parameterization - by way of non-limiting example - allows not only the selection between the single end or differential operating mode, but also capable of allowing to set a bias value to be applied to the biological electrical signals S when acquired by the human-machine interface device 1 may be present.

The second operating unit 120 is designated to control the user interface device 110, such as a device of the Slave EtherCAT type. A slave device is designated to transmit signals and data directly intended for a master controller described hereinafter to the fieldbus. In particular, in the specific case of fieldbuses of the Ethercat type, the communication between slave devices can take place only by passing through a master device. The third operating unit 130 is dedicated to controlling the side of the fieldbus 20, and it is in particular configured to supervise the control of the data signals passing through the fieldbus 20, and it is therefore an EtherCAT interface. In this manner, the third operating unit is connected to (or alternatively it comprises) the aforementioned I/O terminal 14. Advantageously, the use of a hardware structure with mutually separate operating units increases the scalability and/or modularisation of the human-machine interface device 1 described herein.

For the purposes of the present disclosure, the expression BCI signals is used to indicate human-machine interface signals, originating from the central nervous system, in particular at least from the brain, of a subject 13.

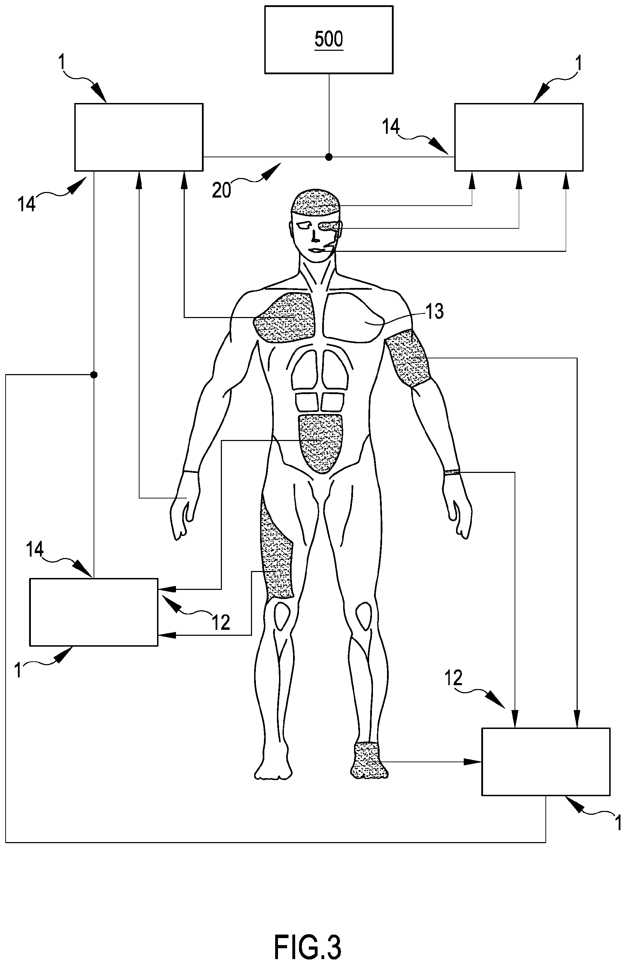

The human-machine interface device 1 subject of the present disclosure is designed for the synchronised reading of signals coming from the human body. Thanks to this aspect it is possible to analyse body signals temporally correlated to each other; processes for evaluating complex phenomena involving the human body therefore become possible by means of the device described herein. Such signals can be of different nature and related to the following functions: brain, electrical, magnetic, temperature, motor activity, cardiac pulsation, as well as of the electrocardiographic type, or for monitoring sleep study, or bispectral analysis, or for evoked potentials or even myographic signals. Figure 3 illustrates a configuration in which exchanged on the fieldbus 20 are data signals 30 coming from a first human-machine interface device suitable to receive a first type of neuromuscular signals (corresponding to biological electrical signals taken from the brain in the figure), from the ocular area and from the area of the mouth of the subject 13, or for example brain and/or craniofacial area signals), a second human-machine interface device suitable to receive a second type of neuromuscular signals (corresponding to biological electrical signals taken from the pectoral area and of the left hand of the subject 13 in the figure), a third human machine interface device suitable to receive a third type of neuromuscular signals (corresponding to biological electrical signals taken from the abdominal and thigh area of the subject 13 in the figure), and a fourth human-machine interface device suitable to receive a fourth type of biological signals (in the figure corresponding to biological electrical signals of the biceps, of the wrist and podal area). Such signals are temporarily synchronised by means of the master controller 500. In particular, in the system subject of the present disclosure, the synchronisation of the various human-machine interface devices 1 connected on the fieldbus 20 can take place according to two alternative modes: a distributed clock mode and a so-called "sync master" mode.

The outer casing of the human-machine interface device 1 subject of the present disclosure has a casing which can be made with different degrees of IP protection, as indicated in the EN 60529 standard (implemented by the IEC as the IEC 70-1 standard), as well as allowing to work in civil, industrial, military and medical environments.

Positioned inside the human machine interface device 1 are different components which can be schematically summarised in three areas: a first area, identified with the reference numeral 470, is dedicated to the data acquisition system of the human body; such first zone 470 is operatively connected to the aforementioned inputs 12 and it is interfaced with sensors 416 external to the device subject of the present disclosure, which can be - by way of non-limiting example - electrodes. A second area 480 comprises the control logic zone and it is operatively connected to the first zone 470.

A third zone 490, or fieldbus interface subsystem, corresponding to the third operating unit 130 of figure 2, is responsible for the synchronisation between, and of, the first area 470 and of the second area 480. The third zone 490 also contains the circuitry that in use allows the connection of the device to the master controller 500 of the network.

In detail, the first zone 470 comprises a front-end 413 configured to receive and, if necessary, amplify the biological signals S coming from the body of the subject 13. Said front end 413, in a preferred and non-limiting embodiment, is configured to quantise the biological electric signal S on a predetermined number of bits, preferably equal to or greater than 12, or preferably equal to or higher than 16 or even more preferably equal to or higher than 20. In a particular embodiment, there is a quantisation of the biological signal S on 24 bits. This quantisation is designed for the purpose of keeping a resolution equal to or less than 0.5µV. The biological signal S reaches the input 12 through connectors 411 of the standard type. In a particular embodiment, such connectors are of the M8 or M12 type. An input-output front-end 414, present in the first zone 470, is designed to provide output signals, preferably of the electrical type, of the synchronised type and/or to read and/or receive trigger signals external to the human-machine interface device 1, preferably provided with a corresponding timestamp.

The data is transmitted from the first zone 470 to the second zone 480 by means of a processor 420 which is directly connected to the second zone 480.

Lastly, the first zone 470 comprises probe terminals 410 which are designed to provide as output, or receive as input, debug signals 415 for controlling the operation of the device 1. This allows to check, by means of an external probe or external instrument, the trend of the electrical signals at least within the first zone 470.

In an embodiment, the first zone 470 is single for each human-machine interface device 1. However, such configuration shall not be deemed as limiting since it is possible to have a human-machine interface device 1 provided with a plurality of first zones 470, preferably though not limited to stack-mounted; this allows the use of several data acquisition modules controlled by a single processor 420. The hardware interface may be compatible with the Arduino header standard, or it can alternatively be of a different type. Thanks to this aspect it is possible to have an extremely versatile device in adapting and interfacing with modules produced by different manufacturers.

The third fieldbus interface zone or subsystem 490 is the zone designed for transmitting and receiving the data signal 30 on the fieldbus 20, in particular to and from the master controller 500. The I/O interface 14 thereof comprises connectors 451, 452 of the standard type for the transmission of data signals which, in a preferred and non-limiting embodiment, are of the NIC 457 type. Such connectors may also be of the RJ45 type and/or have appropriate levels of IP protection. Thanks to this aspect, also from the side of the fieldbus 20, it is possible to have adequate protection against external agents. In a particular embodiment, the I/O interface connectors are of the M8 or M12 type. Furthermore, these connectors can be optical fibre connectors, plastic connectors or made of class. Thanks to this aspect it is possible to install human-machine interface devices 1 that allow to reach up to one kilometre of distance from the master controller 500.

The third fieldbus interface zone or subsystem 490 also comprises a functional block, schematically represented with the reference numeral 454; the purpose of said functional block is to contain electronic data describing profiles and/or movements and/or input/output relative to or electronically associated with the type of subject under examination. The profiles specifically supported by the functional block 454 may regard the motion control such as the DS402, DS301 for I/O or safety profiles, where required. In the latter case, the electronics are revised as a function of the standards required by the application in particular. The same profiles are optional and they can be implemented on the master controller 500 with the block indicated by the reference numeral 541, so as to allow a dialogue between the two electronics always compatible with the standards regarding the different fieldbuses.

It was previously mentioned that the synchronisation of the human-machine interface devices 1 connected on the fieldbus 20 may alternatively occur in a first distributed clock mode, or in a second "sync master" mode. In the sync master mode, the human-machine interface device 1 subject of the present disclosure is configured to receive a data signal 30 through the I/O interface 14, in particular a data signal containing a synchronisation EtherCAT frame. Upon receipt of the aforementioned synchronisation EtherCAT frame, an interrupt signal is generated inside the human-machine interface device 1; upon generation of the interrupt signal and/or in the substantial temporal correspondence thereof, the first operating unit 110 is activated to cause the reception and/or sampling and/or quantisation of the biological electric signal S on the input 12. In other words, in the master sync mode, the biological signal acquisition on the input 12 occurs only at predetermined time instants, which are specifically determined by the generation of the interrupt. Thanks to this aspect it is possible to precisely define the time instant in which the signal is sampled.

The distributed clock mode has a further advantage with respect to the master sync mode: the distributed clock mode causes a simultaneous sampling of biological electrical signals by a plurality of human-machine interface devices 1 connected to each other, which - in the master sync mode - would otherwise sample biological electrical signals at slightly different instants due to their position in the network (in particular in a mode in which, especially in the network configuration of figure 1, the first human-machine interface device 1 starts to sample first with respect to the last human-machine interface device 1). In the distributed clock mode, each human-machine interface device 1 operatively connected to the fieldbus 20, transmits data signals on the fieldbus 20 through the I/O terminal 14 and it receives and/or samples and/or quantises the biological electric signal S at predefined time instants, which are shared and substantially identical for all the human-machine interface devices 1 connected on the fieldbus 20.

To this end, the third fieldbus interface zone or subsystem 490 further comprises a distributed clock synchronisation module 450 configured to receive a clock signal from a master controller 500 external to the human-machine interface device 1. In particular, the clock signal is such to allow a synchronisation precision between several human-machine interface devices 1 in the order of the nanosecond. It should be noted that the level of precision reached in the system subject to the present disclosure is not affected by the number of man-machine interface devices 1 simultaneously connected to the fieldbus 20. The distributed clock synchronisation module 450 in particular it comprises a hardware clock parameterised and remotely controlled by the master controller 500 through the fieldbus 20, in order to allow to generate, at predetermined time instants, an interrupt signal inside the human-machine interface device 1.

The working modes of the distributed clock synchronisation module 450 are indicated by the human-machine interface device 1 and they are also set by the master controller 500 during the network start-up steps; alternatively, these working modes are dynamically modified during the system operation cycles.

The signals generated by the distributed clock synchronisation module 450 may be all those available from the EtherCAT standard, and may preferably but not limited be signals for managing times, offsets, modes and direction.

In an embodiment the synchronisation of the system is not given by the master controller 500, but by a human-machine interface device 1 among the plurality of those connected on the fieldbus 20; preferably, by way of non-limiting example, such human-machine interface device 1 is the first, or closest, to the master controller 500. In a further non-limiting embodiment, the synchronisation of the system occurs by means of an external device, operatively connected on the fieldbus 20, provided with its own hardware or software module for the generation of distributed clock signals. Still alternatively, by way of a non-limiting embodiment, a clock source according to the PTP 1588 standard or in any case to an equivalent precision time protocol can be connected on the fieldbus 20. Advantageously, using a PTP standard allows to carry out a timestamp of each single packet or frame that transits on the fieldbus 20. Although with lower performance, in principle the clock source may use the NTP.

It should be noted that should there be need to extend the acquisition of biological signals S and/or the transceive of data signals 30 on the fieldbus 20 by means of several systems for controlling, for example, several machines 40, the PTP 1588 allows to keep the synchronisation of several systems each provided with a master controller 500, defining a macrosystem 600 each of which comprises a system such as the one described above, wherein each master controller 500 manages - independently from the others - its own system 601 but with a synchronisation of data transceive on the fieldbuses 20 of each system which is totally free. The macrosystem 600 provides a distributed computing environment. In any case, due to the distance that can exist between the machine 40 and the human-machine interface device 1, the assembly of these two devices, or each system 601, can also define a distributed computing environment, i.e. wherein the computational power of the assembly as a whole is distributed on devices located in different areas.

The third fieldbus interface zone or subsystem 490 further comprises a mailbox 453, configured to manage an acyclic-type data transceive; optionally said mailbox 453 carrying out said transceive according to at least one of the standards selected from the present list: CanOpen over EtherCAT, File over EtherCAT, Ethernet over EtherCAT, Safety over EtherCAT, Vendor Specific. In particular, the mailbox 453 allows to exchange data between devices 1 in an acyclic manner on the fieldbus 20. Otherwise, the human-machine interface device 1 which is the subject of the present disclosure can perform a cyclic data exchange on the fieldbus 20, by means of a data processing module 412, always part of the third fieldbus interface zone or subsystem 490, which - on the other hand - is always present.

As mentioned, the second zone 480 provides the logic control unit for managing the synchronisation of the data acquisition subsystem 470 and of the fieldbus interface subsystem 490. The second zone comprises a microprocessor 420, and a real-time clock (RTC) module 428 configured to manage the programming of the real-time actions performed by the logic control unit 480 by defining, for each action, a priority with respect to other actions and/or by defining, for each action, a predefined execution time. The power of the microprocessor 420 must be such that, should the human-machine interface device 1 have several first stack-connected zones 470, the maintenance of the predefined data and signal processing frequency can be kept for each of the first zones 470.

The second zone further comprises a temperature sensor 425 operatively connected to the processor 420 and configured to detect a temperature present in real-time at least inside the human-machine interface device 1. Such temperature sensor may for example cause a reduction in the operating speed of the processor when there is an excessive temperature inside the human-machine interface device 1.

Passing through second zone 480 are various signals, of the electrical type, of the general-purpose input/output (GPIO) 427 type, which can be freely parameterised with digital input and output functions and/or with analogue input/output functions.

A communication interface module, identified with reference numeral 423, allows communication between the various components of the second zone 480 and between the first zone 470 and the third zone 490. In particular, the communication interface module 423 of the second zone 480 manages the internal bus thereof, which - in a preferred and non-limiting embodiment - can be an SPI, QSPI, I2C bus and/or be an 8-bit or 16-bit or 32-bit parallel bus.

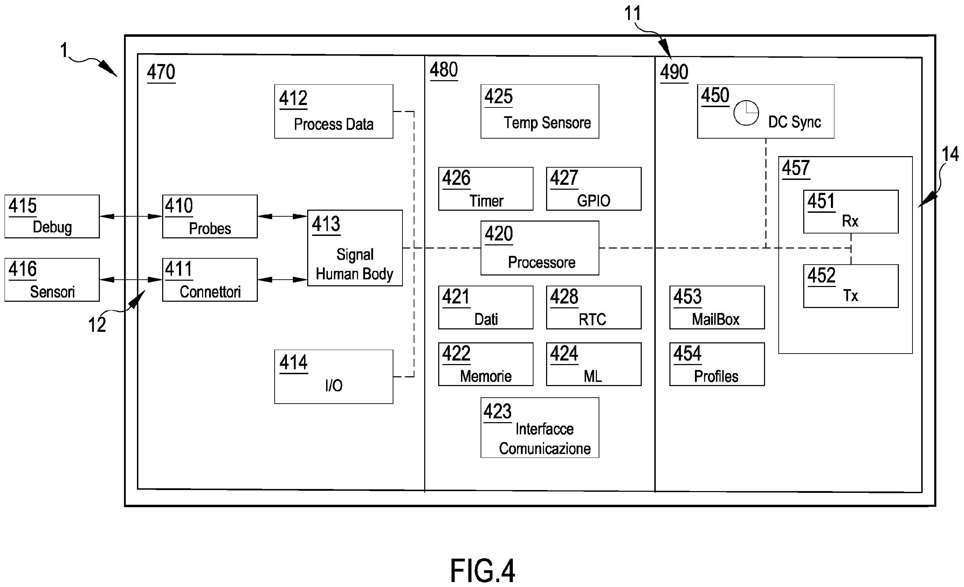

One or more memories 422 are electrically connected at least to the processor 420 and it/they allow to store, even in a non-volatile manner, data (indicated with the reference numeral 421 in figure 4) relating to the operation of the human-machine interface device 1.

The second zone further comprises a timer 426 whose purpose is to manage the prioritisation of the activities carried out by the device and the times with which they are carried out.

The second area or fieldbus interface 480 comprises a machine learning module 424 operatively connected to the processor 420. Such machine learning module 424 is configured to store dedicated learning algorithms that the processor 420 can recall when required to interpret biological signals S coming from the user 13 relating to reactions and/or for controlling the machine 40.

In use, the processor 420 is configured to process the biological electrical signals S received through the input 12 by means of said machine learning module 424 and/or by means of a digital signal processing module (not shown in the attached figures) part of the logic control unit 480.

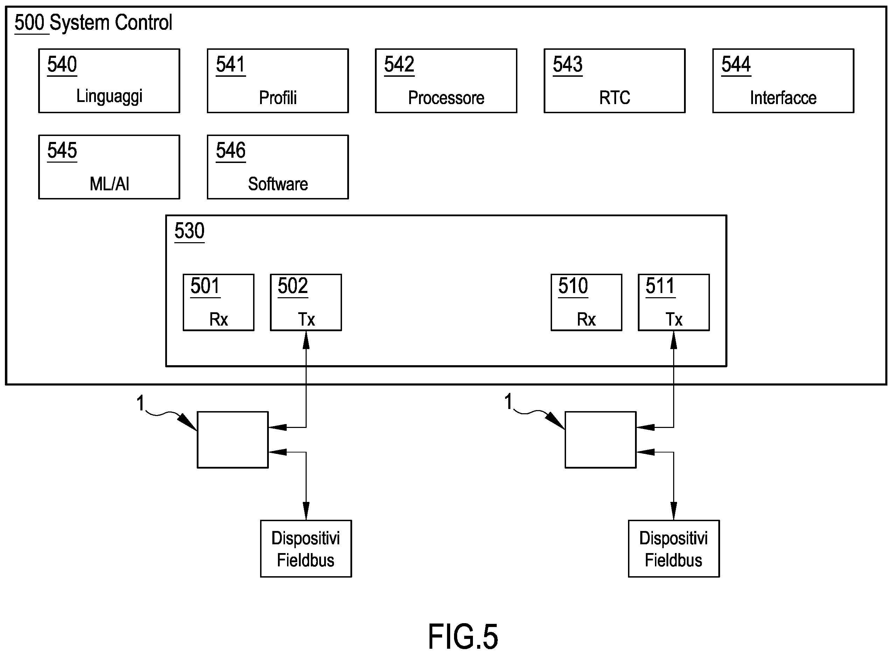

Figure 5 schematically represents the internal of the master controller 500. It is preferably a PC-embedded system, or a PC provided with its own dedicated microcontroller and/or a digital signal processing module. In a preferred and non-limiting embodiment, such master controller 500 may be provided with, or be, a PLC. The master controller 500 integrates real-time scheduling control functions and synchronisation of the management of a fieldbus network, in particular an EtherCAT network. Thanks to this aspect the master controller 500 can supervise the deterministic operation of the system. In this case, the master controller 500 is a controller of the EtherCAT type.

Like in the case of the human-machine interface device 1, the master controller 500 can be provided with a casing suitable for working in particular even in harsh humid and/or dusty environments. Thus, the casing provides an appropriate IP protection against external agents; thanks to this aspect, the master controller can also work in particular environments such as military or civil and industrial.

The master controller 500 accesses the fieldbus 20 by means of an interface subsystem 530 comprising input and output connectors 501, 502, 510, 511 for the transmission and reception of data signals 30 of the packet type, toward and from human-machine interface devices 1.

The master controller 500 comprises a processor 542, which may be a processor provided with a single core or, alternatively, it may be a processor provided with a multiple core. The processor 542 may have an Intel, ARM or AMD type technology and/or it may be or it may comprise an ASIC and/or a PLC.

The master controller 500 further comprises peripherals 544 operatively connected to the processor 542, which - by way of non-limiting example - comprise RAM, PCI, PCIe, ISA, gateway USB, USB ports, hard disk, SSD, MMC and/or modules for wireless data transmission.

Inside a memory operatively accessible by the processor 542, a software or firmware is stored which can be written in a programming language 540 according to the C/C++/IEC PLCOpen standard and/or Matlab and/or according to the IEC61131-3 standard. Such software or firmware program may be used for controlling and/or programming the processor 542 or it can also be dedicated to further applications, such as - by way of non-limiting example - the handling of the data signals 30 transmitted and/or received respectively toward/from the fieldbus 20.

In particular, the master controller 500 comprises a clock signal distribution module 543 which is configured to cause - in use - the transmission of a synchronisation signal (also called distributed clock signal) on the fieldbus 20 toward the various human-machine interface devices 1 which are connected thereto. The clock signal distribution module 543 is operatively connected to the processor 542, and the clock signals are signals contained in a packet or frame, so that they can be read according to the conventional reading and writing standard typical of the fieldbus 20.

In the memory, there is a software or firmware (schematically indicated in figure 5 with the reference numeral 546) which integrates at least one extension for the management of signals in real-time, so as to allow the master controller 500 to manage the determinism of operation of the system.

The master controller 500 also comprises a plurality of profiles identified in figure 5 with the reference numeral 541. Such profiles relate to all the EtherCAT slave devices connected to the fieldbus 20 and represented by the previously described human-machine interface devices 1. In particular, the profiles 541 can be profiles for managing the movement control of the machine 40 and they can be according to the DS402 or DS301 standard. In the profiles 541, there is an ESI configuration file according to the EtherCAT standard from which it is possible to manage the parameterisation of the human-machine interface devices 1 that are the subject of this disclosure. Alternatively, it is possible to manage devices interfaced to the fieldbus 20 without ESI configuration files by reading an EEPROM. Thanks to this aspect it is therefore possible to obtain a computing network in which devices of a heterogeneous nature can be connected on a single fieldbus 20, such as - by way of non-limiting example - EtherCAT modules, drives, robots, vision and/or I/O signals management systems, fieldbus gateways even according to different standards including, for example, ProfiNET, EtherNet/IP, ProfiBUS, CanOpen, DeviceNet.

In a particular embodiment, the master controller 500 comprises a machine learning module 545 which can be advantageously used to spread at least part of the algorithm used during the operation toward the human-machine interface devices 1.

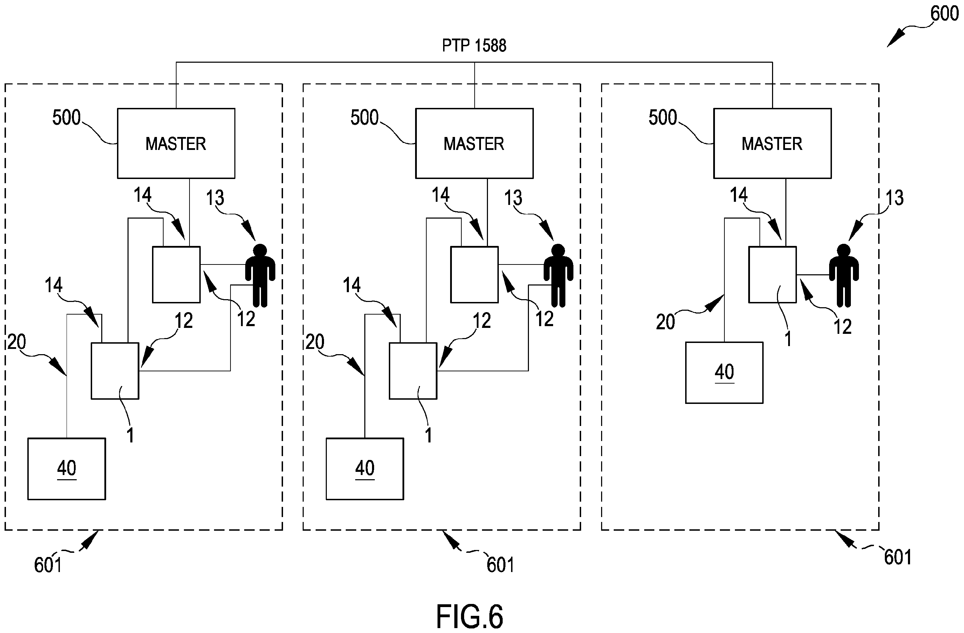

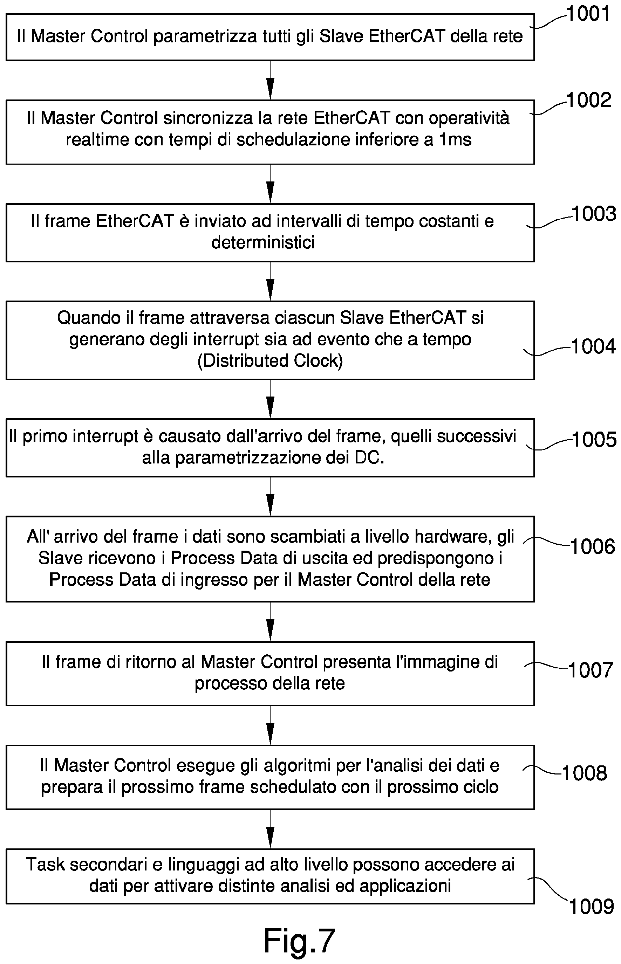

Figure 7 illustrates the main steps performed by the system subject of the present disclosure for each data acquisition cycle. A first step (block 1001) performed by the master controller 500 is that of a parameterization of all the human-machine interface devices 1 which are described as "slave EtherCAT" in the diagram of figure 6. A subsequent step (block 1002) is that of synchronisation of the network so as to be able to operate with real-time operation and with times for transmitting data signals 30 on the fieldbus lower than 1ms. Subsequently, a third step (block 1003) is that of sending a data signal 30 on the fieldbus 20, in particular in the form of EtherCAT frames.

Upon reception of the EtherCAT frame (or more generally of the data signal 30) on a human-machine interface device 1 (block 1004) interrupt signals are generated both event-based and time-based. In particular, the first interrupt signal is caused by the arrival of the frames, while the subsequent ones by the parameterisation of the distributed clock signals (block 1005).

When the EtherCAT frame is received on each human-machine interface device 1, the data are transmitted inside this device via hardware. The other human-machine interface devices 1, which are present on the fieldbus 20, receive data of the output "Process Data" type and they prepare input Process data for the master controller 500 of the network (block 1006).

At this point (block 1007), a human-machine interface device 1 connected on the fieldbus 20 transmits the network process image to the master controller 500, and - subsequently (block 1008) - the master controller 500 automatically performs an own algorithm for the analysis of the data received by means of the image of the network process, and it prepares electronically and automatically a subsequent frame prepared (scheduled) to be sent at the subsequent working cycle.

Secondary tasks and high-level languages can access the data transceived on the fieldbus 20 (block 1009) to activate separate analyses and applications.

By means of the human-machine interface device 1 subject of the present disclosure, it is therefore possible to obtain an interface method between a machine 40 and a subject 13, in which there is - above all - a step, on an input 12 of the human-machine interface device 1, for receiving at least one biological electrical signal S, in particular coming at least from the brain of a subject 13, wherein the biological electrical signal S is optionally captured by means of a sensor 416 positioned on the body of the subject 13.

The method described above comprises a processing step, in which the biological electric signal S received at the input 12 is processed electronically by an electronic circuit 11, electrically connected to the input 12.

Therefore, there is a step for transmitting a data signal 30 comprising the biological electric signal S processed by the electronic circuit 11 and/or based on the biological electric signal S processed by the electronic circuit 11, wherein in said step for transmitting the data signal 30 is transmitted, by means of an I/O terminal 14 of said human-machine interface device 1, directly on the fieldbus 20, in order to be directed toward said machine 40. The processing of said data signal 30 occurs substantially in real time and/or the processing of said data signal 30 occurs with the maintenance of a deterministic and/or speed component in the transmission of the signal on the fieldbus 20.

In this case, the method comprises establishing a logic connection between a master controller 500, configured at least to control a plurality of human-machine interface devices, and at least one human-machine interface device 1; following the establishment of said logic connection, the master controller 500 and the human-machine interface device 1, or if present a plurality of human-machine interface devices 1, form a network interconnected by means of said fieldbus 20; this network also defines a system 601 as described previously.

In the method subject of the present disclosure, a step for the electronic synchronisation of said network, for example comprising an activity carried out by the master controller 500 is performed, after which the network is configured to carry out the transceive substantially in real-time and/or with deterministic component and substantially predefined speed. Alternatively, this activity can be performed by a human-machine interface device 1 or by an external clock source, but still connected on the fieldbus 20.

Following synchronisation, data signals 30 are transmitted on said network and/or on said fieldbus 20, optionally in the form of frames, at constant and deterministic time intervals.

Furthermore, there is a clock distribution step which occurs by configuring and/or programming the master controller 500, and in particular a clock signal distribution module 543 thereof, for performing the transmission of a synchronisation and/or distributed clock signal on the fieldbus 20 toward one or more human-machine interface devices 1 connected thereto.

The Applicant designed a particular embodiment of the system subject of the present disclosure, capable of mapping 36-byte data signals in input and 4-byte signals in output. From analyses and measurements made by the Applicant, it was observed that the configuration of two human-machine interface devices 1 subject of the present disclosure uses a single EtherCAT frame having a length equal to 146 bytes, and the time it took to passed through the entire network is equal to 13.60 µs.

In a different configuration, comprising 128 human-machine interface devices 1, multiple EtherCAT frames are required. However, even if 128 human-machine interface devices are configured on the network, the total cycle time remains less than 500 µs and each frame, optionally excluding the last one (which requires a shorter time), requires 120.32 µs. The last frame does not saturate the available space to reach 500 µs. In total, the time required for all frames is equal to: 120.32 x 3 + 28.3 = 389.26 µs, and the overall number of signals read is equal to 1024 (128x8).

The signals that may be required for reading an electroencephalogram are equal to 256, although a smaller number of signals may be sufficient. Thus, four subjects 13 can be interfaced simultaneously by means of the system subject of the present disclosure. The ratio between signals (and therefore probes for receiving them) and people as described above shall not be deemed as limiting: the Applicant observes that, by way of non-limiting example, 16 people can be analysed with 64 probes, 32 people with 32 probes, 64 people with 16 people.

The invention is not limited to the embodiments illustrated in the drawings. Thus, it is clear that where characteristics mentioned in the claims are followed by reference signs, such reference signs are included for the sole purpose of increasing the intelligibility of the claims, by no means constituting a limitation of the claims in question.

Lastly, it is clear that the subject of the present disclosure can be subject of additions, modifications or variants obvious to a man skilled in the art without departing from the scope of protection provided by the attached claims.

## Claims

1. Human-machine interface device (1), comprising:
- at least one input (12) configured to receive at least one biological electrical signal (S), optionally coming from at least the brain of at least one subject (13),
- an electronic circuit (11), electrically connected to the input (12) configured to electronically process the biological electrical signal (S),
- at least one I/O terminal (14), operatively connected to the electronic circuit (11) and configured at least to transmit a data signal (30) comprising the biological electrical signal (S) processed by the electronic circuit (11) and/or based on the biological electrical signal (S) processed by the electronic circuit (11) toward a machine (40), through a fieldbus (20), and/or configured to receive a data signal (30) from said fieldbus (20),
said human-machine interface device (1) being a device configured to transmit the data signal (30) comprising at least part of said biological electrical signal (S) and to receive a data signal (30) on/from a fieldbus, said electronic circuit (11) and said I/O terminal (14) being configured at least to keep a deterministic component and/or to keep a predetermined speed in the transmission of the data signal (30), in particular of the respective data signals, on the fieldbus (20).

2. Device according to claim 1, wherein the fieldbus is an EtherCAT bus, or it is a Profinet bus, or it is a Sercos III bus, or it is a Powerlink bus, or it is an EtherNet/IP bus, and wherein the biological electrical signal (S) is, or comprises, at least one of the signals from the following list: an electroencephalographic signal, or an electrocardiographic signal, a sleep study monitoring signal, a bispectral analysis signal, an evoked potentials signal.

3. Device according to claim 1 or claim 2, wherein:
- said deterministic component is such that the transceive of data signals on said fieldbus (20) occurs at pre-set time intervals;
- the fieldbus (20) is a data bus specifically designed to allow the distributed control of a machine (40) by several electronic devices, in particular by means of a serial data transceive.

4. Device according to one or more of the preceding claims 1-3, configured to receive, in particular to sample and/or quantise, said biological electric signal (S) on said input (12), optionally for a predetermined and finite period of time, in a first reception mode and based on a distributed clock signal, transmitted on the fieldbus (20), and wherein in said first reception mode the human-machine interface device (1) is configured to compensate for signal propagation delays in said fieldbus (20), and/or it is configured to receive, and/or quantise and/or sample the biological electrical signal (S) at the input (12), optionally for a predetermined and finite period of time, in a second reception mode, by generating an interrupt signal caused by the reception of a data signal (30) on the I/O terminal (14).

5. Device according to one or more of the preceding claims 1-3, configured to operate in a first distributed clock mode in which the transmission of data signals (30) on the I/O terminal (14) and at least the reception, and/or quantisation and/or sampling of the biological electrical signal (S) on the input (12) are performed at predefined time instants based on a clock signal.

6. Device according to claim 4, configured to automatically activate, following the generation of said interrupt signal, an electronic reception and/or sampling of the biological electric signal (S) on said input (12), and/or wherein the human-machine interface device (1) is configured to receive and/or sample the biological electrical signal (S) at at least one predetermined time instant at the generation of said interrupt signal and/or determined by the generation of said interrupt signal.

7. Device according to one or more of the preceding claims, wherein the human-machine interface device (1) is a slave-type device, configured to receive a synchronisation signal from a master controller (500) transmitting the synchronisation signal on the same fieldbus (20) to which the human-machine interface device (1) accesses through the I/O terminal (14), and/or wherein the human-machine interface device (1) is a slave device, configured at least to transmit data and/or signals directly directed to a master controller (500) on said fieldbus (20).

8. Device according to one or more of the preceding claims, configured to sample data signals (30) and/or biological electrical signals (S) at a frequency greater than or equal to 1.0 kHz, preferably greater than or equal to 1.5 kHz, or even more preferably greater than or equal to 2 kHz, or configured to sample data signals (30) and/or biological electrical signals (S) at a frequency greater than or equal to 5 kHz, preferably greater than or equal to 10 kHz, preferably greater than or equal to 15 kHz, preferably up to 20 kHz;
wherein the electronic circuit (11) is configured to sample and/or quantise said biological electrical signal (S) on a number of bits ≥12, preferably ≥16, even more preferably ≥20, even more preferably equal to 24 bits,
and wherein said data signal (30) comprises a quantised biological electrical signal (S) and/or it comprises a biological electrical signal (S) quantised according to a scale of precision greater than or equal to 0.5µV.

9. Device according to one or more of the preceding claims, wherein the electronic circuit (11) comprises at least one first operating unit (110) for managing biological human-machine interface signals, connected to the input (12) and/or comprising the input (12),
the first operating unit (110) being configured to operate in a first single-end mode or in a second and different differential mode and/or to receive the biological electrical signal (S) on the input (12) in a first single-end mode or in a second and different differential mode, and/or wherein the human-machine interface device (1) is configured to operate in a first single-end mode or in a second and different differential mode,
the human-machine interface device (1), in particular the first operating unit (110) comprising a switch and/or a breaker configured at least to allow the alternative selection between said first single-end mode and said second differential mode.

10. Device according to one or more of the preceding claims, wherein the electronic circuit (11) comprises a fieldbus interface subsystem (490), optionally an EtherCAT interface subsystem, configured to manage a serial data stream towards and from the I/O terminal (14), and wherein the fieldbus interface subsystem (490) comprises a mailbox (453), configured to manage an acyclic-type data transceive; optionally said mailbox (453) carrying said transceive according to at least one of the standards selected from the present list: CAnOpen over EtherCAT, File over EtherCAT, Ethernet over Ethercat, Safety over EtherCAT, Vendor Specific.

11. Device according to one or more of the preceding claims,
- comprising a data acquisition subsystem (470) configured to operate as an interface stage toward the plurality of inputs (12);
- comprising a logic control unit (480) operatively connected to the data acquisition subsystem (470), optionally electrically connected to the data acquisition subsystem (470);
- the human-machine interface device (1), in particular the electronic circuit (11), further comprising a fieldbus interface subsystem (490), optionally an EtherCat interface subsystem, configured to manage a serial data flow from and toward the I/O terminal (14);
wherein
- the logic control unit (480) is configured to manage the synchronisation of the data acquisition subsystem (470) and of the fieldbus interface subsystem (490) and/or to manage the synchronisation between the data acquisition subsystem (470) and the fieldbus interface subsystem (490), and
- the logic control unit (480) comprises a microprocessor, and a real-time clock (RTC) module (428) configured to manage the programming of the real-time actions performed by the logic control unit (480) by defining, for each action, priority over other actions and/or by defining, for each action, a predefined run time.

12. A human-machine interface system, said system comprising:
- a master controller (500), configured to at least control a plurality of human-machine interface devices (1) according to one or more of the preceding claims,
- at least one human-machine interface device (1) according to one or more of the preceding claims,
- a fieldbus (20), operatively accessed by the master controller (500) and by the at least one human-machine interface device (1),
wherein the assembly formed by the controller (500) and by the at least one human-machine interface device (1) form a network, and wherein the master controller (500) is configured to synchronise said network, configuring it to carry out the transceive substantially in real time and/or with deterministic component and substantially predefined speed.

13. Human-machine interface method, in particular by means of a human-machine interface device (1), said method comprising:
- a step of receiving, on an input (12) of the human-machine interface device (1), at least one biological electrical signal (S), in particular coming from at least the brain of a subject (13),
- a processing step, wherein the biological electrical signal (S) received on the input (12) is electronically processed by an electronic circuit (11), electrically connected to the input (12),
- a step of transmitting a data signal (30) comprising the biological electrical signal (S) processed by the electronic circuit (11) and/or based on the biological electrical signal (S) processed by the electronic circuit (11), wherein in said transmission step the data signal (30) is transmitted, by means of an I/O terminal (14) of said human-machine interface device (1), directly on a fieldbus (20), in particular an EtherCAT-type fieldbus, so as to be directed towards a machine (40),
wherein the processing of said data signal (30) occurs substantially in real time, and wherein as a result of said transmission step the machine (40) is controlled, performing operations related to said data signal (30) transmitted on the fieldbus (20).

14. Method according to claim 13, wherein the step of receiving the biological electrical signal (S) comprises receiving and/or sampling and/or quantising said biological electrical signal (S) on said input (12), optionally for a predetermined and finite period of time, in a first reception mode and based on a distributed clock signal, transmitted on the fieldbus (20), and wherein in said first reception mode the human-machine interface device (1) is configured to compensate for signal propagation delays in said fieldbus (20),
and/or comprises receiving, and/or quantising and/or sampling, the biological electric signal (S) at the input (12), optionally for a predetermined and finite period of time, in a second reception mode, by generating an interrupt signal caused by the reception of a data signal (30) on the I/O terminal (14).

15. Method according to claim 13 or claim 14, comprising a step of a distribution of a clock signal on the network and/or on the fieldbus (20), so as to electronically synchronise a plurality of human-machine interface devices (1) connected thereto, wherein the clock distribution step occurs by configuring and/or programming the master controller (500), and in particular a clock signal distribution module (543) thereof, for transmitting a synchronisation and/or clock signal distributed on the fieldbus (20) toward one or more human-machine interface devices (1) connected thereto.
